# EUROPEAN PATENT APPLICATION

(11) **EP 4 166 059 A1**
(43) Date of publication of application: **19.04.2023**
(21) Application number: 22200211.5
(22) Date of filing: 07.10.2022
(51) Int. Cl.: A61B 1/00, A61M 25/00

(54) **REINFORCED WORKING CHANNEL TUBE FOR AN ENDOSCOPE**

(30) Priority: 13.10.2021 DE 102021126571
(71) Applicant: Ambu A/S, 2750 Ballerup (DK)
(72) Inventor: MATTHISON-HANSEN, Kaspar Mat, 3000 Helsingør (DK); HANSEN, Michael Kappler, 2665 Vallensbæk Strand (DK)
(74) Representative: Winter, Brandl - Partnerschaft mbB

(57) **Abstract**

The present disclosure relates to a method for producing an at least locally reinforced working channel tube (18) for an endoscope (2), the method comprising the following steps: providing an elongated hollow tube main body (24) initially having an essentially smooth inner surface (26) and an essentially smooth outer surface (28) and comprising a meltable plastic material at least on its outer surface (28); providing a wire (30), preferably made of metal; at least locally heating the wire (30); applying a tensioning on the wire (30) and winding the at least locally heated wire (30) at least in sections around the outer surface (28) of the tube main body (24); softening or melting the outer surface (28) of the tube main body (24) by the wire (30); securely anchoring or embedding the wire (30) in the outer surface (28) of the tube main body (24); and forming a coil (32) anchored or embedded in the outer surface (28) of the tube main body (24) through the winding of the wire (30). Moreover, the present disclosure relates to a working channel tube (18) produced by said method, a use of said working channel tube (18) and an endoscope (2) comprising said working channel tube (18).

## Description

The present disclosure relates to a reinforced working channel tube for an endoscope, a method for producing the working channel tube, a use of the working channel tube and an endoscope comprising the working channel tube.

### Related art

Known endoscopes usually comprise: an endoscope handle or interface comprising a handle or interface housing and a working channel access port; an insertion cord configured to be inserted into a patient's body cavity and comprising an insertion tube, an actively bendable bending section and a distal tip unit; and an internal working channel extending from the working channel access port of the endoscope handle or interface to the distal tip unit of the insertion cord.

Internal working channels are usually formed by a connector part, the so-called biopsy connector or Y-connector, a flexible working channel tube arranged inside the endoscope handle/ interface, the insertion tube and the bending section, and a tip housing of the distal tip unit. A surgical instrument may be guided through the internal working channel into the patient's body cavity, i.e. distally with respect to the tip of the endoscope. Additionally the internal working channel is usually also used as a suction channel to aspirate for example body fluid from an operation area within the patient's body cavity.

In addition to internal working channels there are also already known external working channels which can be mounted to the insertion cord of an endoscope from outside and which may serve as an additional lumen for guiding instruments into the patient's body cavity, for aspirating body fluids from the operation area, etc.

Both internal and external working channels usually comprise a flexible working channel tube having an elongated hollow tube main body usually made of a plastic/ polymer material.

When the actively bendable bending section of the insertion cord is bent, e.g. by manually operating a wheel or lever provided at the endoscope handle/ interface, there is basically the danger that a kinking of the working channel tube occurs due to its insufficient strength/ stability. Kinking usually goes along with a change of the shape of the cross section of the working channel tube. In particular, the cross-sectional shape of the working channel tube is in sections not circular anymore. This may affect a movement of instruments inside the working channel tube and a suction performance.

Therefore, it is basically desirable to reduce or avoid the risk of kinking of the working channel tube during operation of the endoscope, in particular during bending of the actively bendable bending section. It is in this light already known to provide reinforced working channel tubes.

US 6 315 715 B1 e.g. discloses a flexible endoscope with a working channel. The working channel is a flexible tube made from a plastic material and has a convoluted portion in which a wall of the tube is folded to increase flexibility. The working channel tube may be manufactured by placing a helical reinforcing coil in its entirety over a portion of the plastic tube and by axially compressing the helical reinforcing coil and said portion of the tube at room temperature or at an elevated temperature, so that said portion of the tube is folded into a convoluted configuration in which the reinforcing coil is secured by convoluted walls of the compressed plastic tube.

Further, US 4 714 075 A discloses a working channel for a flexible endoscope, wherein the working channel is a thermoplastic tubular member which can be softened by heat. In order to reinforce the tubular member a metal knitted or braided fabric is thermally bonded to the tubular member by moving a heating die over the length of the tubing, softening the outer surface of the tubular member and forcing the reinforcing fabric inwardly into the softened material.

Moreover, US 4 676 229 A discloses an endoscope with a working channel comprising a plastic tube and a metal wire that is helically wound around the plastic tube. The metal wire is bonded to the outer surface of the plastic tube using an adhesive.

In addition, from JP H05-184533 A, JP 4859539 B2 and JP 5189802 B2 it is known to first form or cut a groove on an outer surface of a working channel tube made of plastic material and to afterwards wind a steel wire in the groove so as to form a coil.

It is thus already known from the prior art to provide a reinforced working channel tube having a reinforcing structure like a wire, a fabric or a coil, which are anchored and/or embedded in or on an outer surface of the working channel tube.

Existing reinforced working channel tubes however inter alia have the problem that their method for producing is expensive and requires a plurality of process/ manufacturing steps.

### Brief description of the disclosure

In view of the above-described problem, it is an object of the present disclosure to provide a reinforced working channel tube for an endoscope, which can be simply and cost-effectively produced and which effectively reduces the danger of kinking during its operation.

This object is solved by a method for producing a reinforced working channel tube for an endoscope in accordance with claim 1, by a working channel tube in accordance with claim 7, by a use of the working channel tube in accordance with claim 13 and by an endoscope comprising the working channel tube in accordance with claim 14. Advantageous aspects of the present disclosure are claimed in the dependent claims and/or are described herein below.

The present disclosure relates to a method for producing a reinforced working channel tube for an endoscope, preferably a method for producing a working channel tube, which is reinforced by a coil, the method comprising the following steps: providing an elongated hollow tube main body initially having an essentially smooth inner surface and an essentially smooth outer surface and comprising a meltable plastic material at least on its outer surface; providing a wire, preferably made of metal; at least locally heating the wire; applying a tensioning on the wire and winding the at least locally heated wire at least in sections around the outer surface of the tube main body; softening or melting the outer surface of the tube main body by the wire; securely anchoring or embedding the wire in the outer surface of the tube main body; and forming a coil anchored or embedded in the outer surface of the tube main body through the winding of the wire.

It is to be understood that the reinforced working channel tube produced by said method may be a working channel tube which is entirely reinforced, i.e. over its entire length, or which is only reinforced in sections, over only a portion of its entire length, i.e. only locally.

Moreover, it is to be understood that the essentially smooth inner and outer surfaces of the elongated hollow tube main body relate to inner and outer shell surfaces of the tube main body, which preferably has a round cross-section. Essentially smooth inner and outer surfaces are to be understood as surfaces which have a surface roughness usually obtained in a manufacturing process in which the elongated hollow tube main body is manufactured, e.g. in an extrusion process, preferably without post-processing. Said differently, essentially smooth surfaces are preferably to be understood as unmachined surfaces, which do not comprise grooves, threads, etc.

Further, it is to be understood that the elongated hollow tube main body is preferably a flexible tube. It is to be understood that the feature that at least an outer surface of the elongated hollow tube main body is made of a meltable plastic material means that the elongated hollow tube main body may be entirely made of a meltable plastic material or only/ at least an outer surface of the same may be made of a meltable plastic material. A meltable plastic material is to be understood as plastic/ polymer/ synthetic material which can melt or soften/ which is capable of melting or softening and which preferably has a melting point or range/ area. In particular, plastic materials having thermoplastic behavior like thermoplastics, thermoplastic elastomers, etc. are to be understood as meltable plastic materials.

In addition, it is to be understood that "at least locally heating the wire" means that only a local part/ portion of the wire may be heated or that the wire may be heated in its entirety.

Furthermore, it is to be understood that "winding the at least locally heated wire at least in sections around the outer surface of the tube main body" means that the wire may be wound around the elongated hollow tube main body only locally, i.e. a portion of the tube main body, or that the wire may be wound around the tube main body along the entire length of the tube main body.

It is to be understood that "embedding the wire in the outer surface of the tube main body" means that the wire may be covered at least in sections or completely by the meltable plastic material of the tube main body after the production of the working channel tube. I.e. the melted plastic material may flow over the wire and may close a gap/ groove created through the winding of the wire around the tube main body, so that from outside at least in sections or completely the wire is not visible any more since the wire is fixed firmly and deeply in a surrounding mass of the tube main body.

Further, it is to be understood that "anchoring the wire in the outer surface of the tube main body" means that the wire may be (alternatively or additionally) in sections or completely only anchored and thus not necessarily be fully/ completely covered by the material of the tube main body after the production of the working channel tube. This means that when the wire is anchored in the outer surface of the tube main body then it is still visible from outside. Preferably, at least 50% of a thickness of the wire are anchored in the outer surface of the tube main body for achieving a secure anchoring.

It is thus within the scope of the present disclosure that the wire is covered by the material of the tube main body, i.e. is embedded in the material of the tube main body, i.e. is not visible from outside anymore after production of the working channel tube and that the wire is not covered by the material of the tube main body, i.e. is anchored in the material of the tube main body, i.e. is visible from outside after production of the working channel tube.

In other words, the method for producing a reinforced working channel tube for an endoscope according to the present disclosure may be described such that the (metal) wire/ thread is wound around the elongated hollow tube main body and during the winding process, the wire is heated to a specific temperature and is tensioned so that the outer, initially smooth surface of the tube main body melts/ softens and the wire cuts into the outer surface and is embedded or anchored in the outer surface/ an outer layer of the tube main body. Said differently, during the winding thermal energy/ heat is added to the wire in an amount allowing the wire to soften the outer surface of the tube main body made of the meltable plastic material, enabling a secure anchoring or embedding of the wire in the outer surface of the tube main body.

The method for producing a reinforced working channel tube for an endoscope according to the present disclosure provides a number of advantages. As the wire is anchored or embedded in the outer surface of the tube main body, the outer diameter of the tube main body is essentially unchanged or only slightly increased, which is advantageous in particular in the light of the limited available space inside an insertion cord of an endoscope. Moreover, the method preferably does not affect the inner surface of the tube main body, which preferably remains unchanged and smooth, or does only insignificantly affect the inner surface of the tube main body. A small deformation by the coil/ small ripples on the inner surface/ the inside of the tube main body may be acceptable as long as tools may be advanced through the working channel essentially without increased friction. The method makes it possible that the wire is securely anchored or embedded in the outer surface/ outer layer of the tube main body and reinforces the working channel tube. Thus, the wire forms a reinforcing coil and prevents or at least reduces a danger of kinking of the working channel tube. The wire is anchored or embedded in the outer surface/ layer of the tube main body during winding without requiring any further production steps. There is no need to heat the tube main body or to push the wire into the outer surface of the tube main body afterwards. The wire is inserted/ embedded/ anchored into the tube main body from the outside essentially through the winding tension and its temperature and it is not necessary according to the present disclosure to provide grooves in the outer surface of the tube main body before winding the wire around the same. The winding is thus preferably performed directly on the smooth outer surface of the tube main body. The method according to the present disclosure is thus simple, cost-effective and adaptable to specific purposes. Compared to prior art methods for producing reinforced working channel tubes several complicated process steps may be eliminated.

The method preferably comprises the step: using the working channel tube having the securely anchored or embedded wire in the outer surface of the tube main body, thereby forming the coil, as part of an internal or external working channel of an endoscope.

Preferably, the method further comprises the step: creating a specific or desired pitch of the coil, preferably between 0.5 mm and 1.5 mm, especially preferred around 1 mm. Especially preferred the method comprises the steps: turning the tube main body around its longitudinal axis; axially moving the tensioned wire (in particular a wire providing device like a winch) along the longitudinal axis of the tube main body; and creating a specific or desired pitch of the coil, preferably between 0.5 mm and 1.5 mm, especially preferred around 1 mm, by correspondingly setting a (rotational) speed of the tube main body and an axial movement velocity of the tensioned wire.

Turning the tube main body around its longitudinal axis may allow winding the wire around the tube main body without having to move the wire in a circumferential direction. A spindle may for example turn the tube main body. By controlling the axial movement of the wire/ the wire providing device, the pitch of the coil may be set or adjusted as requested. The wire may move in the axial direction and may be stationary in a circumferential direction of the tube main body. This may lead to a cost-effective production of the reinforced working channel tube. For example, a winch may be provided to wind the wire around the tube main body. By appropriately adjusting/ setting the pitch a reinforcement-degree of the working channel tube may be set. It is noted that the reinforcement-degree of the tube main body may alternatively or additionally be adjusted by changing a cross-sectional shape or area of the wire.

It is to be understood that the present disclosure is not limited to the embodiment according to which the tube main body is turned around its longitudinal axis and the tensioned wire is axially moved along the longitudinal axis of the tube main body. The tube main body e.g. may also be stationary/ fixed and the wire may be moved both axially and circumferentially with respect to the longitudinal axis of the tube main body. Further alternatively, the wire may be held stationary, i.e. may not move axially if the tube main body is both rotated and axially moved. Also with these alternative embodiments, a specific or desired pitch of the coil may be appropriately set.

Preferably, the method further comprises the step: heating the wire only locally immediately before winding the locally heated wire around the smooth outer surface of the tube main body. When the wire is heated only locally immediately before winding the same around the tube main body it may be ensured that occurring heat losses are reduced/ minimized and an energy-efficient production/ manufacturing method is provided. Said differently, it is preferred to heat the wire only at a portion of the same, which is immediately afterwards wound around the tube main body, so that said portion may cut into and melt the outer surface/ layer of the tube main body easily and effectively. The wire may e.g. be heated by contact heating, preferably by a soldering iron. Contact heating is preferred according to the present disclosure as it may produce less heat losses compared to contactless heating. The use of the soldering iron is e.g. a cost-effective way to heat the wire. However, it is to be understood that any heating method/ means may be used/ provided as long as it enables the wire to be locally or entirely heated.

Preferably, the method further comprises the step: tensioning the wire between 0.1 N and 10 N, preferably between 0.5 N and 3 N, especially preferred around 2 N, during the winding. This may ensure - together with the heating - that the wire may be securely anchored or embedded in the outer surface/ layer of the tube main body. The tensioning (force) may be set such that the wire does not cut entirely through the tube main body. Controlling the tensioning of the wire may thus make it possible to control how deep the wire cuts into the outer surface of the tube main body. It is to be understood that the tensioning force applied is of course dependent on properties/ materials of the tube main body and the wire and other tensioning forces are basically conceivable.

Preferably, the method further comprises the step: winding the wire only in sections around the outer surface of the tube main body, i.e. not along an entire length of the tube main body, thus providing an only locally reinforced working channel tube. The properties of the reinforced working channel tube may be varied by reinforcing only sections of the working channel tube. The wire may e.g. be added only in a local area, for example an area of the working channel tube which is arranged in/ inside an actively bendable bending section of an insertion cord of an endoscope. Thereby the coil formed by the wound wire may locally improve the kinking resistance and may ensure that a circular cross section of the working channel tube is kept during bending. By providing local reinforcement a working channel tube having varying properties along its length may be produced. As the reinforcement according to the present disclosure preferably does not affect or does only insignificantly affect the smooth inner surface of the tube main body, movement behavior of surgical instruments through the working channel and suction performance are not impaired by providing only local reinforcement of the working channel tube.

Preferably, the method further comprises the step: providing the elongated hollow tube main body with an outer layer comprising the outer surface and an inner layer comprising the inner surface, wherein a material of the outer layer is different from a material of the inner layer. When the two layers, i.e. the outer layer and the inner layer, are made of different material, each layer may be optimized for its specific purpose. The outer layer of the tube main body is made of a meltable plastic material that is configured to melt or soften when it is heated according to the present disclosure, thus allowing the hot wire to melt or soften the outer layer and cut into the same when it is wound around the tube main body. The inner layer may be made of a meltable plastic material, however does not have to be made of the same, as it is not intended to be melted or softened. The inner layer and thus its material may be optimized for other purposes, e.g. enabling a smooth gliding of an instrument inside the working channel tube. The outer layer may e.g. be made of TPU (thermoplastic polyurethane) and the inner layer may be made of HDPE (high-density polyethylene).

According to a preferred embodiment, the materials of the outer layer and the inner layer may both be meltable plastic material and a melting temperature or range/ area of the material of the inner layer may be higher than a melting temperature or range/ area of the material of the outer layer. This makes it possible to adjust a temperature of the heated wire such that said temperature is above the melting temperature/ range/ area of the material of the outer layer and below the melting temperature/ range/ area of the material of the inner layer. This preferred embodiment makes it possible that the wire cannot melt or soften the inner layer, as its temperature is not high enough. A cut-in deepness of the wire may thus be appropriately defined, essentially independently from the tensioning force applied to the wire.

The inner layer may be thinner than the outer layer, or said differently, a wall thickness of the inner layer may be smaller than a wall thickness of the outer layer. E.g. the inner layer may have a wall thickness of between 0.050 mm to 0.080 mm and the outer layer may have a wall thickness of between 0.32 mm to 0.35 mm. However, other wall thicknesses are conceivable and it is also conceivable that the wall thickness of the inner layer is greater than the wall thickness of the outer layer.

Optionally, a primer may be provided between the inner layer and the outer layer to ensure and increase an adherence between the two layers. This is e.g. of advantage if the outer layer is made of TPU (thermoplastic polyurethane) and the inner layer is made of HDPE (high-density polyethylene).

According to an alternative preferred embodiment the tube main body may also be an integral part made of only one meltable plastic material, thus not comprising the outer layer and inner layer made of different materials. In this case the tensioning force and the temperature of the wire should be suitable set (e.g. by performing experiments) such that the wire does not cut through the entire wall thickness of the tube main body.

The method preferably comprises the step: fixing an end of the wire to the tube main body after forming the coil.

Preferably, the wire is broken off after completion of the winding, e.g. by bending the wire multiple times in different directions. It may be especially preferred if a sharp bend, in particular a kink, is provided to the wire at the outer surface of the tube main body before bending the wire back and forth multiple times in different directions. E.g. the end of the wire may be pushed against the tube main body, in particular against the outer surface of the tube main body, at a specific position, and the sharp bend of the wire may be provided at said specific position. The sharp bend is preferably performed such that a weakness point or starting point for breaking off the wire is provided on the outer surface of the tube main body or at least very close to the outer surface of the tube main body so that the wire is preferably broken flush with the outer surface of the tube main body when the wire is subsequently bent back and forth multiple times in different directions. The sharp bend can e.g. be manually provided by pushing the wire against the specific position by a fingernail, or automatically by a pushing or pressing body. Breaking the wire flush with the outer surface of the tube main body provides the advantage that a risk of a sharp wire end sticking out of the outer surface of the tube main body is reduced, thereby reducing a potential risk of damaging other components of the endoscope, such as a camera wire, other tubes, etc., in particular in the assembly process. Alternatively, the wire may be cut after termination of the winding process.

The risk of the wire end sticking out of the outer surface of the tube main body may be further reduced, in particular after breaking off the wire by the sharp bend as mentioned above, by adding a drop of adhesive to the wire end, thereby covering the wire end with adhesive, and fixing the wire end to the tube main body. The end of the wire may be additionally or alternatively anchored or embedded in the tube main body after the winding is completed, e.g. by locally heating and melting the tube main body and pressing the end of the wire into the locally heated and melted portion of the tube main body. Thus, the end of the wire may be fixed to the tube main body. Said differently, the method preferably also comprises the step: fixing a free end of the wire to the tube main body by locally heating and melting a portion of the tube main body and pressing the free end of the wire into said portion of the tube main body.

The present disclosure may also relate to a method for producing an endoscope comprising the method for producing the reinforced working channel tube as described above and a step of introducing/ assembling the reinforced working channel tube having the securely anchored or embedded wire in the outer surface of the tube main body, thereby forming the coil, into an endoscope, e.g. as part of an internal or external working channel.

The present disclosure also relates to a working channel tube comprising: an elongated hollow tube main body, preferably having a round cross section, having an essentially smooth inner surface and comprising a meltable plastic material at least on its outer surface; and a coil, preferably made of metal, firmly attached and anchored or embedded in the outer surface of the tube main body, the coil being formed by: providing a wire; at least locally heating the wire; applying a tensioning on the wire and winding the at least locally heated wire at least in sections around an initially smooth outer surface of the tube main body; softening or melting the outer surface of the tube main body by the wire; and securely anchoring or embedding the wire in the outer surface of the tube main body.

Expressed in other words, the working channel tube comprises the elongated hollow tube main body and the (metal) reinforcing coil anchored or embedded in the outer surface of the tube main body. The reinforcing coil is formed by winding the (metal) wire around the tube main body. During the winding, thermal energy/ heat is added to the wire in an amount allowing the wire to soften the outer surface of the tube main body made of the meltable plastic material, enabling a secure anchoring or embedding of the wire in the outer surface of the tube main body. The working channel tube according to the present disclosure is thus manufactured applying a specific process having a small number of production steps and being cost-effective. Said process makes it possible to at least locally/ in sections reinforce the working channel tube, thus making the reinforced working channel tube less vulnerable to kinking. The working channel tube may e.g. be reinforced in an area, which is intended to be arranged in an actively bendable bending section of an endoscope.

The working channel tube having the coil firmly attached and anchored or embedded in the outer surface of the tube main body is preferably (sterile) packaged in a (sterile) packaging, i.e. the working channel tube is preferably a (sterile) packaged working channel tube. This has the advantage that the working channel tube may be independently manufactured and stored, and may be introduced/ assembled into an endoscope as part of an internal or external working channel anytime thereafter.

Preferably, the inner diameter of the working channel tube is greater than 2 mm and smaller than 4.5 mm. Kinking is a problem that may depend on the inner diameter of the working channel tube, and it has been found that tubes having an inner diameter of 2 mm or less may not have the problem of kinking. However, it is nevertheless conceivable to also reinforce such small-diameter working channel tubes having an inner diameter of less than 2 mm. Because of a limited outer diameter of an insertion cord of the endoscope and a limited space inside the insertion cord, the inner diameter of the tube main body is preferably less than 4.5 mm. A preferred inner diameter of the tube main body is around 3.8 mm according to the present disclosure.

Preferably, a pitch of the coil is between 0.5 mm and 1.5 mm, especially preferred around 1 mm.

Preferably, the elongated hollow tube main body comprises an outer layer comprising the outer surface and an inner layer comprising the inner surface, wherein a material of the outer layer is different from a material of the inner layer. Especially preferred, the materials of the outer layer and of the inner layer are both meltable plastic materials and a melting temperature of the material of the inner layer is higher than a melting temperature of the material of the outer layer.

Preferably, the outer diameter of the reinforced working channel tube is essentially the same as the outer diameter of the elongated hollow tube main body. As the wire is preferably completely anchored or embedded in the outer surface of the tube main body, the wire preferably "disappears" in the tube main body, i.e. preferably does not protrude radially from the outer surface of the tube main body. The outer diameter of the reinforced tube main body is thus preferably not increased or only increased by a very small amount compared to the initial tube main body. This may be advantageous when the reinforced working channel tube is arranged inside an insertion cord of an endoscope, where only limited space is available.

Preferably, the coil/ the wire is made of metal. Especially preferred, the coil/ wire is made of steel, e.g. stainless steel. However, any other metal materials like copper, titanium, etc. are also conceivable. Moreover, also non-metal materials are conceivable for the coil/ the wire as long as the wire made of said material can be appropriately heated and tensioned.

The wire used to form the coil preferably has a round cross-section, however flat wires may basically also be used.

Preferably, the wall thickness of the tube main body is at least two times greater than a diameter or thickness (in case of a flat wire) of the wire, so that the wire can be suitably anchored or embedded in the outer surface of the tube main body. E.g., the wall thickness of the tube main body may be between 0.3 mm and 0.5 mm, e.g. around 0.4 mm and the diameter/ thickness of the wire may be between 0.08 mm and 0.15 mm. Other wall thicknesses and wire diameters/ thicknesses may be used as long as the wall thickness of the tube main body is at least two times greater than the diameter or thickness of the wire.

The present disclosure also relates to a use of a working channel tube as described above as part of an internal or external working channel of an endoscope.

Further, the present disclosure relates to an endoscope comprising: an endoscope handle or interface comprising a handle or interface housing and a working channel access port; an insertion cord configured to be inserted into a patient's body cavity and comprising an insertion tube, an actively bendable bending section and a distal tip unit; and a working channel extending from the working channel access port of the endoscope handle or interface to the distal tip unit of the insertion cord and comprising a working channel tube as described above.

Preferably, the working channel tube extends through the bending section and is locally reinforced by the coil (only) in the bending section.

### Brief description of figures

The disclosure is explained in more detail below using preferred embodiments and referring to the accompanying figures.
- Fig. 1: shows a schematic view of an endoscope according to the present disclosure.
- Fig. 2: shows a flow chart of a method for producing a working channel tube according to the present disclosure.
- Fig. 3: shows the working channel tube according to the present disclosure during its production.
- Fig. 4: shows an arrangement of a tube main body in a winding apparatus.
- Fig. 5: shows a cross-section through a tube main body according to an embodiment of the present disclosure.
- Fig. 6: is a longitudinal sectional view of a working channel tube having a coil embedded in a tube main body according to an embodiment of the present disclosure.
- Fig. 7: is a longitudinal sectional view of a working channel tube having a coil anchored in a tube main body according to an embodiment of the present disclosure.

The figures are schematic in nature and serve only to understand the disclosure. The features of the different embodiments can be interchanged among each other.

### Detailed description of preferred embodiments

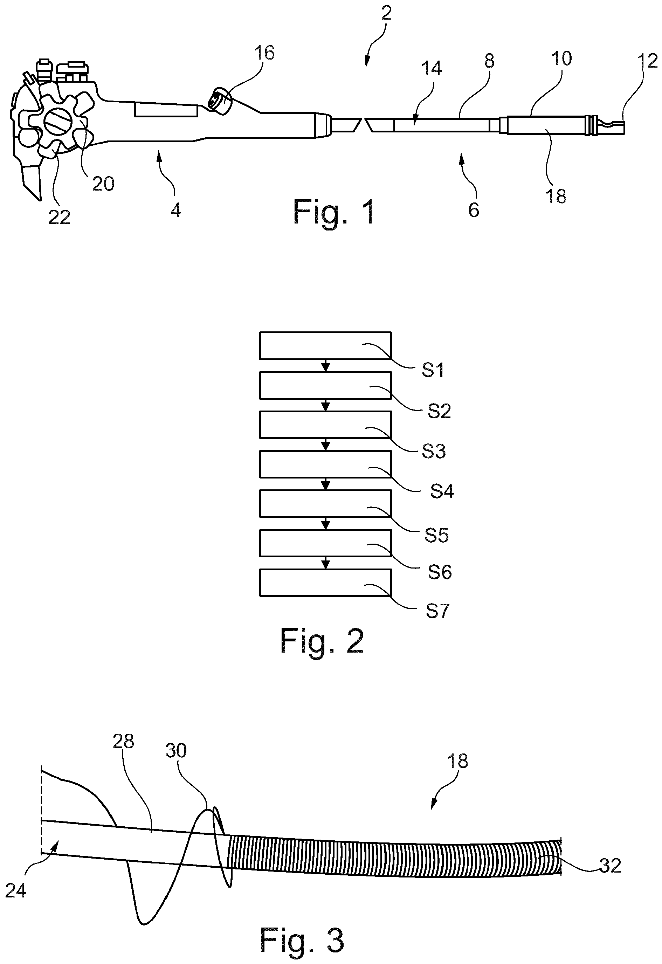

Fig. 1 shows a schematic view of an endoscope 2, which is preferably a single-use endoscope. The endoscope 2 has a proximal endoscope handle 4 and an insertion cord 6 extending distally from the endoscope handle 4. The insertion cord 6 is configured to be inserted into a patient's body cavity and comprises an insertion tube 8, an actively bendable bending section 10 and a distal tip unit 12. The endoscope 2 further comprises a working channel 14 which extends from a working channel access port 16 provided at the endoscope handle 4 to the distal tip unit 12. The working channel 14 comprises a working channel tube 18, which is arranged inside the endoscope handle 4, the insertion tube 8 and the bending section 10. The endoscope handle 4 comprises two operating units 20, 22 formed as wheels for steering the bending section 10 of the insertion cord 6. In particular, a rotation/ turning force can be applied to both the first operating unit 20 and the second operating unit 22 by a user in order to bend the bending section 10 in two bending planes/ four directions. The endoscope 2 may alternatively be formed as a one-plane bending endoscope which can be bent only in two, preferably opposite, directions and which has only one operating unit 20, 22.

The working channel tube 18 provided inside the endoscope handle 4, the insertion tube 8 and the bending section 10 is a specific reinforced working channel tube 18 according to the present disclosure and is in particular reinforced in a portion of the working channel tube 18 which is arranged inside of the bending section 10, in order to prevent kinking of the working channel tube 18 when the bending section 10 is bent. It is to be understood that it is also conceivable to reinforce the entire working channel tube 18, i.e. not only the portion of the working channel tube 18 which is arranged inside the bending section 10 of the insertion cord 6, or other portions of the working channel tube 18. Further, it is to be understood that the working channel tube 18 does not need to be arranged inside the insertion cord 6 but may alternatively also be attached to the insertion cord 6 from outside.

With reference to Fig. 2, Fig. 3 and Fig. 4 a method for producing a reinforced working channel tube 18 according to the present disclosure is described. Fig. 2 shows a flow chart of the method for producing a reinforced working channel tube 18. In a first step S1 an elongated hollow tube main body 24 initially having an essentially smooth inner surface 26 and an essentially smooth outer surface 28 and comprising a meltable plastic material at least on its outer surface 28 is provided. In a step S2 a wire 30, preferably made of metal, is provided. The wire 30 is at least locally heated in a step S3. In a step S4 a tensioning is applied on the wire 30 and the at least locally heated wire 30 is at least in sections directly wound around the smooth outer surface 28 of the tube main body 24. The heated wire 30 softens or melts the outer surface 28 of the tube main body 24 in a step S5. In a further step S6 the wire is securely anchored or embedded in the outer surface 28 of the tube main body 24. In a step S7 a coil 32 anchored or embedded in the outer surface 28 of the tube main body 24 is formed through the winding of the wire 30. It is to be understood that the process steps S4 to S7 are performed simultaneously/ follow immediately one after another. In particular, when the at least locally heated wire 30 is wound around the smooth outer surface 28 of the tube main body 24, it is a direct consequence of the temperature of the wire 30 and the tension applied on the wire 30 that the wire 30 softens/ melts the outer surface 28 of the tube main body 24. Moreover, a direct consequence of the softening/ melting of the outer surface 28 of the tube main body is the secure anchoring/ embedding of the wire 30 in the outer surface 28 of the tube main body 24. The formation of the coil 32 is the direct consequence of the winding and anchoring/ embedding.

Fig. 3 shows the working channel tube 18 for the endoscope 2 during production. The working channel tube 18 comprises the elongated hollow tube main body 24 and the wire 30. The wire 30 is wound around the tube main body 24 and during the winding process, the wire 30 is locally heated to a specific temperature and is tensioned, e.g. by a tensioning force of around 2 N, so that the smooth outer surface 28 of the tube main body 24 melts and the wire 30 cuts into and is embedded/ anchored in the outer surface 28 of the tube main body 24. The wire 30 is preferably a metal wire, e.g. a steel thread, in particular stainless steel. Moreover, the wire preferably has a round cross-section and a wire diameter is e.g. 0.1 mm. The wound wire 30 forms a reinforcing coil 32 at least in sections around the tube main body 24. The winding of the wire 30 is performed such that a specific desired pitch may be reached, which serves to adjust a reinforcement-degree. E.g. the pitch can be set such that it is between 0.5 mm and 1.5 mm, e.g. around 1 mm. The reinforcement-degree may additionally or alternatively be adjusted by appropriately setting a cross-sectional area of the wire 30. The tube main body 24 may have an inner diameter (defined by the inner surface 26) of around 3.8 mm. A wall thickness of the tube main body 24 may e.g. be 0.4 mm.

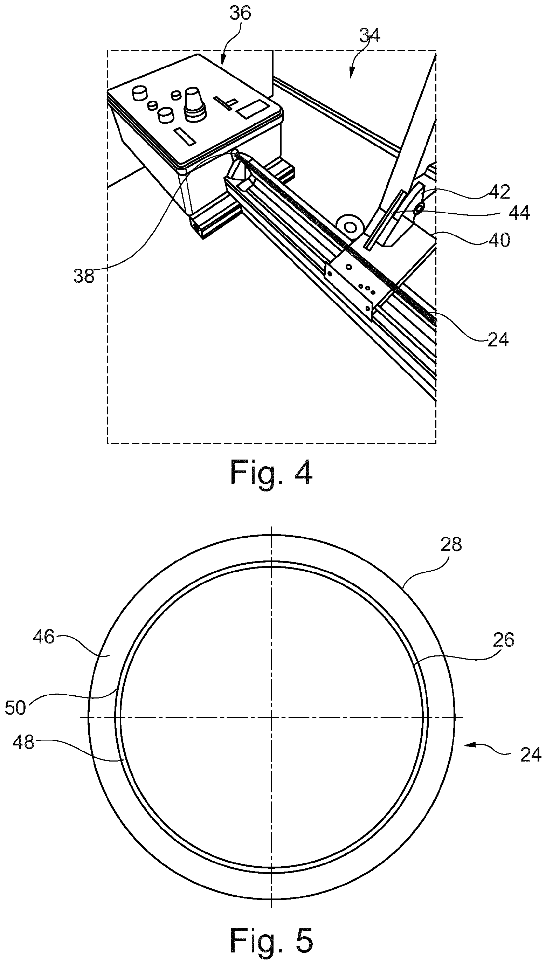

Fig. 4 shows an arrangement of the elongated hollow tube main body 24 in a winding apparatus 34. The winding apparatus 34 comprises a control unit 36, a spindle 38, a slide 40, a winch 42 and a soldering iron 44. The tube main body 24 is mounted to the spindle 38 and may be rotated by the spindle 38. The winch 42 and the soldering iron 44 are fixedly connected to the slide 40, which may be displaced/ moved in an axial direction of the tube main body 24. The control unit 36 controls parameters of the production process, e.g. the axial movement velocity of the slide 40 or the rotational speed of the spindle 38 or a tensioning force applied on the wire 30. The winch 42 may provide the wire 30. The wire 30 is locally heated by the soldering iron 44 and is then directly wound around the tube main body 24. The winding is performed by rotating the tube main body 24 around its longitudinal axis by the spindle 38 and by axially moving the slide 40 and the winch 42 (and thus the wire 30) along the longitudinal axis of the tube main body 24 according to this preferred embodiment.

Fig. 5 shows a cross-section through a tube main body 24 according to an embodiment of the present disclosure. The tube main body 24 has a circular cross-section and comprises two layers, namely an outer layer 46 and an inner layer 48. Both the outer layer 46 and the inner layer 48 are essentially hollow elongated tube bodies and are preferably made of different materials. The outer layer 46 is provided directly around the inner layer 48, or said differently the inner layer 48 is inserted into the outer layer 46. A primer 50 may be provided between the outer layer 46 and the inner layer 48 in order to ensure adherence between the outer layer 46 and the inner layer 48. E.g. the outer layer 46 may be made of thermoplastic polyurethane (TPU) and the inner layer 48 may be made of high-density polyethylene (HDPE).

The materials used for the outer layer 46 and the inner layer 48 may also be chosen such that they are both meltable plastic materials and a melting temperature of the material of the inner layer 48 is higher than a melting temperature of the material of the outer layer 46. In this case, the temperature of the wire 30 can be adjusted such that it is between the melting temperature of the material of the outer layer 46 and the melting temperature of the material of the inner layer 48.

The embodiment shown in Fig. 5 is only an example and it is evident that it may be also advantageous if the tube main body 24 is not made of two parts (the outer layer 46 and the inner layer 48), but is made of one integral elongated tube main body part having the outer surface 28 and the inner surface 26 and being made of a meltable plastic material.

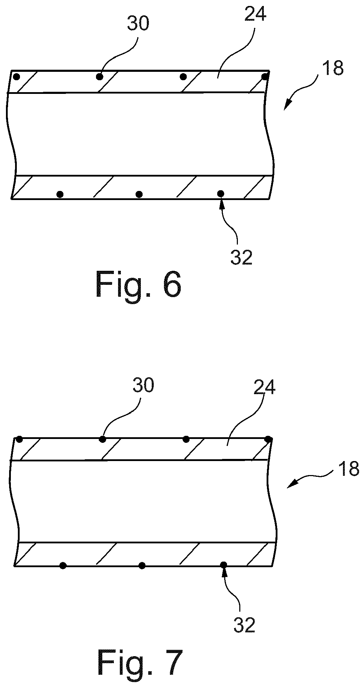

Embodiments according to which the tube main body 24 is made of one integral part are shown in Fig. 6 or in Fig. 7. Fig. 6 shows a longitudinal sectional view of a working channel tube 18 having a coil 32 embedded in a tube main body 24. The wire 30 forming the coil 32 is covered by a material of the tube main body 24 and is thus not visible from outside, since the melted plastic material has flown over the wire 30/ has closed a gap or groove created through the winding of the wire 30 around the tube main body 24 during the production process. Fig. 7 shows a longitudinal sectional view of a working channel tube 18 having a coil 32 anchored in a tube main body 24. The wire 30 forming the coil 32 is not covered by the material of the tube main body 24 after the production of the working channel tube 18 and is thus still visible from outside.

According to an especially preferred embodiment, the tube main body 24 may have an outer diameter of 5.4 ± 0.076 mm, an inner diameter of 4.4 ± 0.076 mm and a tube wall thickness of 0.5 mm. The total length of the tube main body 24 may be 1500 mm according to the especially preferred embodiment. The tube main body 24 may comprise an outer layer 46, an inner layer 48 and a primer 50, which can also be designated as tie layer, between the outer layer 46 and the inner layer 48 according to the especially preferred embodiment. The material of the outer layer 46 may be a thermoplastic polyurethane like Pellethane^{®} 2363-90AE with additives according to the especially preferred embodiment. E.g., the outer layer 46 may comprise 70% Pellethane^{®} 2363-90AE and 30% additives. Among additives, it may be preferred if the outer layer 46 comprises an antimicrobial additive like BaSo4 and a friction reducing additive like ProPell^{®}. The material of the inner layer 48 may be a high-density polyethylene like Borealis Bormed^{®} HE9621-PH. The material of the primer 50 or tie layer may be a linear low-density polyethylene based tie resin like Orevac^{®} 18300M.

The wire 30 may have an outer diameter of 0.15 mm according to the especially preferred embodiment. The material of the wire may be stainless steel according to the especially preferred embodiment, e.g. SUS304. The wire made of SUS304 may comprise a Br-Ni-Co coating.

The pitch of the coil 32 formed through the winding of the wire 30 may be 1 mm according to the especially preferred embodiment. The length of the section of the tube main body 24, which is reinforced by the coil 32, may be 115 ± 5 mm according to the especially preferred embodiment. The section reinforced by the coil 32 may have an inner diameter, which is greater than 4.2 mm, and an outer diameter, which is smaller than 5.4 mm.

The kinking performance of the tube main body 24 according to the especially preferred embodiment with and without reinforcement by the coil 32 has been tested. In particular, bending diameters of 23 mm, 30 mm, 40 mm, 50 mm and 60 mm have been tested. It has shown that the tube main body 24 without reinforcement by the coil 32 does kink for bending diameters of 23 mm, 30 mm and 40 mm and does not kink for bending diameters of 50 mm and 60 mm. The tube main body 24 with reinforcement by the coil 32 does only kink for a bending diameter of 23 mm and does not kink for bending diameters of 30 mm, 40 mm, 50 mm and 60 mm. Therefore, it has shown that according to the present disclosure kinking performance of the working channel tube 18 may be improved.

### List of reference signs

- 2: endoscope
- 4: endoscope handle
- 6: insertion cord
- 8: insertion tube
- 10: bending section
- 12: distal tip unit
- 14: working channel
- 16: working channel access port
- 18: working channel tube
- 20: first operating unit
- 22: second operating unit
- 24: tube main body
- 26: inner surface
- 28: outer surface
- 30: wire
- 32: coil
- 34: winding apparatus
- 36: control unit
- 38: spindle
- 40: slide
- 42: winch
- 44: soldering iron
- 46: outer layer
- 48: inner layer
- 50: primer

## Claims

1. A method for producing a reinforced working channel tube (18) for an endoscope (2), the method comprising the following steps:
providing an elongated hollow tube main body (24) initially having an essentially smooth inner surface (26) and an essentially smooth outer surface (28) and comprising a meltable plastic material at least on its outer surface (28);
providing a wire (30), preferably made of metal;
at least locally heating the wire (30);
applying a tensioning on the wire (30) and winding the at least locally heated wire (30) at least in sections around the outer surface (28) of the tube main body (24);
softening or melting the outer surface (28) of the tube main body (24) by the wire (30);
securely anchoring or embedding the wire (30) in the outer surface (28) of the tube main body (24); and
forming a coil (32) anchored or embedded in the outer surface (28) of the tube main body (24) through the winding of the wire (30).

2. The method according to claim 1, comprising further the steps:
turning the tube main body (24) around its longitudinal axis;
axially moving the tensioned wire (30) along the longitudinal axis of the tube main body (24); and
creating a specific or desired pitch of the coil (32), preferably between 0.5 mm and 1.5 mm, especially preferred around 1 mm, by correspondingly setting a rotational speed of the tube main body (24) and an axial movement velocity of the wire (30).

3. The method according to claim 1 or 2, comprising further the step:
heating the wire (30) only locally immediately before winding the locally heated wire (30) around the smooth outer surface (28) of the tube main body (24).

4. The method according to any one of claims 1 to 3, comprising further the step:
tensioning the wire (30) between 0.1 N and 10 N, preferably between 0.5 N and 3 N, especially preferred around 2 N, during the winding.

5. The method according to any one of claims 1 to 4, further comprising the step:
winding the wire (30) only in sections around the outer surface (28) of the tube main body (24), i.e. not along an entire length of the tube main body (24), thus providing an only locally reinforced working channel tube (18).

6. The method according to any one of claims 1 to 5, comprising further the step:
providing the elongated hollow tube main body (24) with an outer layer (46) comprising the outer surface (28) and an inner layer (48) comprising the inner surface (26), wherein a material of the outer layer (46) is different from a material of the inner layer (48).

7. A working channel tube (18) comprising:
an elongated hollow tube main body (24) having an essentially smooth inner surface (26) and comprising a meltable plastic material at least on its outer surface (28); and
a coil (32), preferably made of metal, firmly attached and anchored or embedded in the outer surface (28) of the tube main body (24), the coil (32) being formed by:
providing a wire (30);
at least locally heating the wire (30);
applying a tensioning on the wire (30) and winding the at least locally heated wire (30) at least in sections around an initially smooth outer surface (28) of the tube main body (24);
softening or melting the outer surface (28) of the tube main body (24) by the wire (30); and
securely anchoring or embedding the wire (30) in the outer surface (28) of the tube main body (24).

8. The working channel tube (18) according to claim 7, wherein the tube main body (24) has an inner diameter between 2 mm and 4.5 mm.

9. The working channel tube (18) according to claim 7 or 8, wherein a pitch of the coil (32) is between 0.5 mm and 1.5 mm, especially preferred around 1 mm.

10. The working channel tube (18) according to any one of the claims 7 to 9, wherein the elongated hollow tube main body (24) comprises an outer layer (46) comprising the outer surface (28) and an inner layer (48) comprising the inner surface (26), wherein a material of the outer layer (46) is different from a material of the inner layer (48).

11. The working channel tube (18) according to claim 10, wherein the materials of the outer layer (46) and the inner layer (48) are both meltable plastic materials and a melting temperature of the material of the inner layer (48) is higher than a melting temperature of the material of the outer layer (46).

12. The working channel tube (18) according to any one of the claims 7 to 11, wherein a wall thickness of the elongated hollow tube main body (24) is at least two times greater than a diameter or thickness of the wire (30).

13. Use of a working channel tube (18) according to any one of claims 7 to 12 as part of an internal or external working channel (14) of an endoscope (2).

14. An endoscope (2) comprising:
an endoscope handle (4) or interface comprising a handle or interface housing and a working channel access port (16);
an insertion cord (6) configured to be inserted into a patient's body cavity and comprising an insertion tube (8), an actively bendable bending section (10) and a distal tip unit (12);
a working channel (14) extending from the working channel access port (16) of the endoscope handle (4) or interface to the distal tip unit (12) of the insertion cord (6) and comprising the working channel tube (18) according to any one of claims 7 to 12.

15. The endoscope (2) according to claim 14, wherein the working channel tube (18) extends through the bending section (10) and is locally reinforced by the coil (32) in the bending section (10).
